# EUROPEAN PATENT APPLICATION

(11) **EP 1 239 035 A1**
(43) Date of publication of application: **11.09.2002**
(21) Application number: 00981667.9
(22) Date of filing: 13.12.2000
(51) Int. Cl.: C12N 15/12, C12N 5/10, C07K 14/47, C07K 16/18, A61K 45/00, A61P 21/00, A01K 67/027

(54) **NOVEL PROTEIN AND GENE**

(30) Priority: 17.12.1999 JP 35924399
(71) Applicant: Mitsubishi Pharma Corporation, Osaka-shi, Osaka 541-0046 (JP)
(72) Inventor: SOBUE, Gen, R. 407, Higashiyama-East A, Nagoya-shi, Aichi 468-0002 (JP); DOUYU, Manabu, Room 201, Moriyama-ku, Nagoya-shi, Aichi 463-00 27 (JP)
(74) Representative: Godemeyer, Thomas, Dr.
(86) International application number: JP0008793
(87) International publication number: WO01044468

(57) **Abstract**

Novel proteins involved in amyotrophic lateral sclerosis and genes encoding the same are provided by a protein having an amino acid sequence shown in SEQ ID NO:1 or SEQ ID NO:2, or an amino acid sequence having deletion, substitution or insertion of one or plural amino acids in said amino acid sequences, a gene encoding this protein, an expression vector comprising this gene, a transformant transformed with this expression vector, an antibody against this protein, a method for screening a compound for treatment of a neuro-degenerative disease, a kit for the screening, and a transgenic animal wherein this gene is introduced or deleted.

SEQUENCE LISTING

<110> Mitsubishi-Tokyo Pharmaceuticals, Inc.

<120> NOVEL PROTEINS AND GENES

<130> 00027WO0

<150> JP 11-359243
<151> 1999-12-17

<160> 6

<210> 1
<211> 838
<212> PRT
<213> Homo sapiens

<400> 1
Met Gln Glu Gln Glu Ile Gly Phe Ile Ser Lys Tyr Asn Glu Gly Leu
1               5                   10                  15

Cys Val Asn Thr Asp Pro Val Ser Ile Leu Thr Ser Ile Leu Asp Met
            20                  25                  30

Ser Leu His Arg Gln Met Gly Ser Asp Arg Asp Leu Gln Ser Ser Ala
        35                  40                  45

Ser Ser Val Ser Leu Pro Ser Val Lys Lys Ala Pro Lys Lys Arg Arg
    50                  55                  60

Ile Ser Ile Gly Ser Leu Phe Arg Arg Lys Lys Asp Asn Lys Arg Lys
65                  70                  75                  80

Ser Arg Glu Leu Asn Gly Gly Val Asp Gly Ile Ala Ser Ile Glu Ser

                85                  90                  95

Ile His Ser Glu Met Cys Thr Asp Lys Asn Ser Ile Phe Ser Thr Asn
            100                 105                 110

Thr Ser Ser Asp Asn Gly Leu Thr Ser Ile Ser Lys Gln Ile Gly Asp
        115                 120                 125

Phe Ile Glu Cys Pro Leu Cys Leu Leu Arg His Ser Lys Asp Arg Phe
    130                 135                 140

Pro Asp Ile Met Thr Cys His His Arg Ser Cys Val Asp Cys Leu Arg
145                 150                 155                 160

Gln Tyr Leu Arg Ile Glu Ile Ser Glu Ser Arg Val Asn Ile Ser Cys
                165                 170                 175

Pro Glu Cys Thr Glu Arg Phe Asn Pro His Asp Ile Arg Leu Ile Leu
            180                 185                 190

Ser Asp Asp Val Leu Met Glu Lys Tyr Glu Glu Phe Met Leu Arg Arg
        195                 200                 205

Trp Leu Val Ala Asp Pro Asp Cys Arg Trp Cys Pro Ala Pro Asp Cys
    210                 215                 220

Gly Tyr Ala Val Ile Ala Phe Gly Cys Ala Ser Cys Pro Lys Leu Thr
225                 230                 235                 240

Cys Gly Arg Glu Gly Cys Gly Thr Glu Phe Cys Tyr His Cys Lys Gln
                245                 250                 255

Ile Trp His Pro Asn Gln Thr Cys Asp Ala Ala Arg Gln Glu Arg Ala
            260                 265                 270

Gln Ser Leu Arg Leu Arg Thr Ile Arg Ser Ser Ser Ile Ser Tyr Ser

275                 280                 285

Gln Glu Ser Gly Ala Ala Ala Asp Asp Ile Lys Pro Cys Pro Arg Cys
    290                 295                 300

Ala Ala Tyr Ile Ile Lys Met Asn Asp Gly Ser Cys Asn His Met Thr
305                 310                 315                 320

Cys Ala Val Cys Gly Cys Glu Phe Cys Trp Leu Cys Met Lys Glu Ile
                325                 330                 335

Ser Asp Leu His Tyr Leu Ser Pro Ser Gly Cys Thr Phe Trp Gly Lys
            340                 345                 350

Lys Pro Trp Ser Arg Lys Lys Lys Ile Leu Trp Gln Leu Gly Thr Leu
        355                 360                 365

Val Gly Ala Pro Val Gly Ile Ala Leu Ile Ala Gly Ile Ala Ile Pro
    370                 375                 380

Ala Met Ile Ile Gly Ile Pro Val Tyr Val Gly Arg Lys Ile His Asn
385                 390                 395                 400

Arg Tyr Glu Gly Lys Asp Val Ser Lys His Lys Arg Asn Leu Ala Ile
                405                 410                 415

Ala Gly Gly Val Thr Leu Ser Val Ile Val Ser Pro Val Val Ala Ala
            420                 425                 430

Val Thr Val Gly Ile Gly Val Pro Ile Met Leu Ala Tyr Val Tyr Gly
        435                 440                 445

Val Val Pro Ile Ser Leu Cys Arg Ser Gly Gly Cys Gly Val Ser Ala
    450                 455                 460

Gly Asn Gly Lys Gly Val Arg Ile Glu Phe Asp Asp Glu Asn Asp Ile

465                 470                 475                 480

Asn Val Gly Gly Thr Asn Thr Ala Val Asp Thr Thr Ser Val Ala Glu
                485                 490                 495

Ala Arg His Asn Pro Ser Ile Gly Glu Gly Ser Val Gly Gly Leu Thr
            500                 505                 510

Gly Ser Leu Ser Ala Ser Gly Ser His Met Asp Arg Ile Gly Ala Ile
        515                 520                 525

Arg Asp Asn Leu Ser Glu Thr Ala Ser Thr Met Ala Leu Ala Gly Ala
    530                 535                 540

Ser Ile Thr Gly Ser Leu Ser Gly Ser Ala Met Val Asn Cys Phe Asn
545                 550                 555                 560

Arg Leu Glu Val Gln Ala Asp Val Gln Lys Glu Arg Tyr Ser Leu Ser
                565                 570                 575

Gly Glu Ser Gly Thr Val Ser Leu Gly Thr Val Ser Asp Asn Ala Ser
            580                 585                 590

Thr Lys Ala Met Ala Gly Ser Ile Leu Asn Ser Tyr Ile Pro Leu Asp
        595                 600                 605

Lys Glu Gly Asn Ser Met Glu Val Gln Val Asp Ile Glu Ser Lys Pro
    610                 615                 620

Ser Lys Phe Arg His Asn Ser Gly Ser Ser Ser Val Asp Asp Gly Ser
625                 630                 635                 640

Ala Thr Arg Ser His Ala Gly Gly Ser Ser Ser Gly Leu Pro Glu Gly
                645                 650                 655

Lys Ser Ser Ala Thr Lys Trp Ser Lys Glu Ala Thr Ala Gly Lys Lys

            660                 665                 670

Ser Lys Ser Gly Lys Leu Arg Lys Lys Gly Asn Met Lys Ile Asn Glu
        675                 680                 685

Thr Arg Glu Asp Met Asp Ala Gln Leu Leu Glu Gln Gln Ser Thr Asn
    690                 695                 700

Ser Ser Glu Phe Glu Ala Pro Ser Leu Ser Asp Ser Met Pro Ser Val
705                 710                 715                 720

Ala Asp Ser His Ser Ser His Phe Ser Glu Phe Ser Cys Ser Asp Leu
                725                 730                 735

Glu Ser Met Lys Thr Ser Cys Ser His Gly Ser Ser Asp Tyr His Thr
            740                 745                 750

Arg Phe Ala Thr Val Asn Ile Leu Pro Glu Val Glu Asn Asp Arg Leu
        755                 760                 765

Glu Asn Ser Pro His Gln Cys Ser Ile Ser Val Val Thr Gln Thr Ala
    770                 775                 780

Ser Cys Ser Glu Val Ser Gln Leu Asn His Ile Ala Glu Glu His Gly
785                 790                 795                 800

Asn Asn Gly Ile Lys Pro Asn Val Asp Leu Tyr Phe Gly Asp Ala Leu
                805                 810                 815

Lys Glu Thr Asn Asn Asn His Ser His Gln Thr Met Glu Leu Lys Val
            820                 825                 830

Ala Ile Gln Thr Glu Ile
        835

<210> 2

<211> 219
<212> PRT
<213> Homo sapiens

<400> 2
Met Glu Ala Pro Gly Ala Pro Pro Arg Thr Leu Thr Trp Glu Ala Met
1               5                   10                  15

Glu Gln Ile Arg Tyr Leu His Glu Glu Phe Pro Glu Ser Trp Ser Val
            20                  25                  30

Pro Arg Leu Ala Glu Gly Phe Asp Val Ser Thr Asp Val Ile Arg Arg
        35                  40                  45

Val Leu Lys Ser Lys Phe Leu Pro Thr Leu Glu Gln Lys Leu Lys Gln
    50                  55                  60

Asp Gln Lys Val Leu Lys Lys Ala Gly Leu Ala His Ser Leu Gln His
65                  70                  75                  80

Leu Arg Gly Ser Gly Asn Thr Ser Lys Leu Leu Pro Ala Gly His Ser
                85                  90                  95

Val Ser Gly Ser Leu Leu Met Pro Gly His Glu Ala Ser Ser Lys Asp
            100                 105                 110

Pro Asn His Ser Thr Ala Leu Lys Val Ile Glu Ser Asp Thr His Arg
        115                 120                 125

Thr Asn Thr Pro Arg Arg Arg Lys Gly Arg Asn Lys Glu Ile Gln Asp
    130                 135                 140

Leu Glu Glu Ser Phe Val Pro Val Ala Ala Pro Leu Gly His Pro Arg
145                 150                 155                 160

Glu Leu Gln Lys Tyr Ser Ser Asp Ser Glu Ser Pro Arg Gly Thr Gly

                165                 170                 175

Ser Gly Ala Leu Pro Ser Gly Gln Lys Leu Glu Glu Leu Lys Ala Glu
            180                 185                 190

Glu Pro Asp Asn Phe Ser Ser Lys Val Val Gln Arg Gly Arg Glu Phe
        195                 200                 205

Phe Asp Ser Asn Gly Asn Phe Leu Tyr Arg Ile
    210                 215

<210> 3
<211> 4357
<212> DNA
<213> Homo sapiens

<220>
<221> CDS
<222> (318)..(2831)

<400> 3
atagtcacca gaagctggaa gagtcaaagg acacattctc ccctcaagcc ccagtgggag 60

cacggcccag ctggattttg gacttctggc ctccagaact agacagggcc tcacggtgtc 120

acccagggtg gaatacagtg gtgtgatcat agctcactgc agcctggaat tcctgggctc 180

aagcaaccct gccacctcag ccttccaagt agctaggact acagaacatc catgatagca 240

gtcttctgta aatcgaactt ttcaagaatt ctctgaagga accaagtagg atattcttac 300

atcatgactt aatgtga atg caa gaa caa gaa ata ggt ttt atc tct aaa 350
Met Gln Glu Gln Glu Ile Gly Phe Ile Ser Lys
1               5                   10

tat aat gaa ggg ctg tgt gta aac act gac cct gtc tca att cta aca 398
Tyr Asn Glu Gly Leu Cys Val Asn Thr Asp Pro Val Ser Ile Leu Thr
            15                  20                  25

agc att tta gac atg agt tta cat cgg caa atg ggt tca gat cga gat 446
Ser Ile Leu Asp Met Ser Leu His Arg Gln Met Gly Ser Asp Arg Asp
        30                  35                  40

ctt cag tcc tct gct tca tct gtg agc ttg cct tca gtc aaa aag gca 494
Leu Gln Ser Ser Ala Ser Ser Val Ser Leu Pro Ser Val Lys Lys Ala
    45                  50                  55

ccc aaa aaa aga aga att tca ata ggc tcc ctg ttt cgg agg aaa aaa 542
Pro Lys Lys Arg Arg Ile Ser Ile Gly Ser Leu Phe Arg Arg Lys Lys
60                  65                  70                  75

gat aac aaa cgt aaa tca agg gag cta aat ggc ggg gtg gat gga att 590
Asp Asn Lys Arg Lys Ser Arg Glu Leu Asn Gly Gly Val Asp Gly Ile
                80                  85                  90

gca agt att gaa agt ata cat tct gaa atg tgt act gat aag aac tcc 638
Ala Ser Ile Glu Ser Ile His Ser Glu Met Cys Thr Asp Lys Asn Ser
            95                  100                 105

att ttc tct aca aat acc tct tct gac aat gga tta act tcc atc agc 686
Ile Phe Ser Thr Asn Thr Ser Ser Asp Asn Gly Leu Thr Ser Ile Ser
        110                 115                 120

aaa caa att gga gac ttc ata gag tgc cct ttg tgc ctt ttg cgg cat 734
Lys Gln Ile Gly Asp Phe Ile Glu Cys Pro Leu Cys Leu Leu Arg His
    125                 130                 135

tct aaa gac aga ttt cct gat ata atg act tgt cat cac aga tct tgt 782
Ser Lys Asp Arg Phe Pro Asp Ile Met Thr Cys His His Arg Ser Cys
140                 145                 150                 155

gtg gat tgc tta cga caa tat tta agg ata gaa atc tct gaa agc aga 830
Val Asp Cys Leu Arg Gln Tyr Leu Arg Ile Glu Ile Ser Glu Ser Arg
                160                 165                 170

gtt aat att agt tgc cca gaa tgt act gaa cgg ttt aat ccc cat gat 878
Val Asn Ile Ser Cys Pro Glu Cys Thr Glu Arg Phe Asn Pro His Asp
            175                 180                 185

att cgc ttg ata tta agt gat gat gtc ttg atg gaa aaa tac gaa gaa 926
Ile Arg Leu Ile Leu Ser Asp Asp Val Leu Met Glu Lys Tyr Glu Glu
        190                 195                 200

ttt atg ctt aga cgg tgg ctt gtt gca gat cct gat tgt agg tgg tgt 974
Phe Met Leu Arg Arg Trp Leu Val Ala Asp Pro Asp Cys Arg Trp Cys
    205                 210                 215

cca gct cca gac tgt gga tat gct gtg ata gca ttt gga tgt gcc agc 1022
Pro Ala Pro Asp Cys Gly Tyr Ala Val Ile Ala Phe Gly Cys Ala Ser
220                 225                 230                 235

tgt cca aaa tta act tgt ggg cga gag ggc tgt gga aca gag ttt tgc 1070
Cys Pro Lys Leu Thr Cys Gly Arg Glu Gly Cys Gly Thr Glu Phe Cys
                240                 245                 250

tac cac tgt aaa cag att tgg cac ccc aac cag acc tgt gat gct gct 1118
Tyr His Cys Lys Gln Ile Trp His Pro Asn Gln Thr Cys Asp Ala Ala
            255                 260                 265

cga caa gag aga gcc cag agc tta cgt ttg aga act ata cgt tct tca 1166
Arg Gln Glu Arg Ala Gln Ser Leu Arg Leu Arg Thr Ile Arg Ser Ser
        270                 275                 280

tcc att agt tat agt caa gag tct gga gca gca gct gat gat ata aag 1214
Ser Ile Ser Tyr Ser Gln Glu Ser Gly Ala Ala Ala Asp Asp Ile Lys
    285                 290                 295

cca tgt cca cga tgt gct gct tat ata ata aag atg aat gat ggg agc 1262
Pro Cys Pro Arg Cys Ala Ala Tyr Ile Ile Lys Met Asn Asp Gly Ser
300                 305                 310                 315

tgc aat cac atg aca tgt gct gtt tgt ggt tgt gag ttt tgt tgg ttg 1310
Cys Asn His Met Thr Cys Ala Val Cys Gly Cys Glu Phe Cys Trp Leu
                320                 325                 330

tgt atg aaa gaa atc tca gat ttg cat tat cta agt cca tca gga tgt 1358
Cys Met Lys Glu Ile Ser Asp Leu His Tyr Leu Ser Pro Ser Gly Cys
            335                 340                 345

act ttt tgg ggg aag aaa ccc tgg agc cga aag aag aaa ata ttg tgg 1406
Thr Phe Trp Gly Lys Lys Pro Trp Ser Arg Lys Lys Lys Ile Leu Trp
        350                 355                 360

caa ctg gga aca ctg gtt ggt gct cct gtc gga atc gct tta ata gct 1454
Gln Leu Gly Thr Leu Val Gly Ala Pro Val Gly Ile Ala Leu Ile Ala
    365                 370                 375

ggc att gct att cct gca atg att att ggc att cct gtg tat gtg ggc 1502
Gly Ile Ala Ile Pro Ala Met Ile Ile Gly Ile Pro Val Tyr Val Gly
380                 385                 390                 395

cgc aag att cac aat cgc tat gaa ggc aag gat gtt tca aag cac aaa 1550
Arg Lys Ile His Asn Arg Tyr Glu Gly Lys Asp Val Ser Lys His Lys
                400                 405                 410

cgg aat ttg gcc ata gca ggt ggt gta acg ttg tct gta atc gtg tct 1598
Arg Asn Leu Ala Ile Ala Gly Gly Val Thr Leu Ser Val Ile Val Ser
            415                 420                 425

cca gta gta gct gca gtg act gta ggt atc ggt gtt cct att atg tta 1646
Pro Val Val Ala Ala Val Thr Val Gly Ile Gly Val Pro Ile Met Leu
        430                 435                 440

gct tat gtc tat ggc gta gtt cca att tct ctt tgt cga agc gga ggt 1694
Ala Tyr Val Tyr Gly Val Val Pro Ile Ser Leu Cys Arg Ser Gly Gly
    445                 450                 455

tgt gga gtc tca gca ggc aat gga aaa gga gtt agg att gaa ttt gat 1742
Cys Gly Val Ser Ala Gly Asn Gly Lys Gly Val Arg Ile Glu Phe Asp
460                 465                 470                 475

gat gaa aat gat ata aat gtt ggt gga act aac aca gct gta gac aca 1790
Asp Glu Asn Asp Ile Asn Val Gly Gly Thr Asn Thr Ala Val Asp Thr
                480                 485                 490

aca tca gta gca gaa gca aga cac aac cca agc ata ggg gag gga agt 1838
Thr Ser Val Ala Glu Ala Arg His Asn Pro Ser Ile Gly Glu Gly Ser
            495                 500                 505

gtt ggt ggg ctg act ggc agt ttg agt gca agt gga agc cac atg gat 1886
Val Gly Gly Leu Thr Gly Ser Leu Ser Ala Ser Gly Ser His Met Asp
        510                 515                 520

cga ata gga gcc atc cga gac aac ctg agt gaa acg gcc agc acc atg 1934
Arg Ile Gly Ala Ile Arg Asp Asn Leu Ser Glu Thr Ala Ser Thr Met
    525                 530                 535

gca cta gct gga gcc agt ata acg ggg agt ctg tca gga agt gcc atg 1982
Ala Leu Ala Gly Ala Ser Ile Thr Gly Ser Leu Ser Gly Ser Ala Met
540                 545                 550                 555

gta aac tgt ttt aac agg ttg gaa gta caa gca gat gta cag aaa gaa 2030
Val Asn Cys Phe Asn Arg Leu Glu Val Gln Ala Asp Val Gln Lys Glu
                560                 565                 570

cgg tac agt cta agt gga gaa tct ggc aca gtc agc ttg gga aca gtt 2078
Arg Tyr Ser Leu Ser Gly Glu Ser Gly Thr Val Ser Leu Gly Thr Val
            575                 580                 585

agt gat aat gcc agc acc aaa gca atg gca gga tcc att ctg aat tcc 2126
Ser Asp Asn Ala Ser Thr Lys Ala Met Ala Gly Ser Ile Leu Asn Ser
        590                 595                 600

tac atc cca ttg gac aaa gaa ggc aac agt atg gag gtg caa gta gat 2174
Tyr Ile Pro Leu Asp Lys Glu Gly Asn Ser Met Glu Val Gln Val Asp
    605                 610                 615

att gag tca aag cca tcc aaa ttc agg cac aac agt gga agc agt agt 2222
Ile Glu Ser Lys Pro Ser Lys Phe Arg His Asn Ser Gly Ser Ser Ser
620                 625                 630                 635

gtg gat gat ggc agt gcc acc cga agt cat gct ggc ggt tca tcc agt 2270
Val Asp Asp Gly Ser Ala Thr Arg Ser His Ala Gly Gly Ser Ser Ser
                640                 645                 650

ggc ttg cct gaa ggt aaa tct agt gcc acc aag tgg tcc aaa gaa gca 2318
Gly Leu Pro Glu Gly Lys Ser Ser Ala Thr Lys Trp Ser Lys Glu Ala
            655                 660                 665

aca gca ggg aaa aaa tca aaa agt ggt aaa ctg agg aaa aag ggt aac 2366
Thr Ala Gly Lys Lys Ser Lys Ser Gly Lys Leu Arg Lys Lys Gly Asn
        670                 675                 680

atg aag ata aat gag acg aga gag gac atg gat gca cag ttg tta gaa 2414
Met Lys Ile Asn Glu Thr Arg Glu Asp Met Asp Ala Gln Leu Leu Glu
    685                 690                 695

caa caa agc acg aac tca agt gaa ttt gag gct cca tcc ctc agt gac 2462
Gln Gln Ser Thr Asn Ser Ser Glu Phe Glu Ala Pro Ser Leu Ser Asp
700                 705                 710                 715

agt atg cct tct gta gca gat tct cac tct agt cat ttt tct gaa ttt 2510
Ser Met Pro Ser Val Ala Asp Ser His Ser Ser His Phe Ser Glu Phe
                720                 725                 730

agt tgt tct gac cta gaa agc atg aaa act tct tgt agt cat ggt tcc 2558
Ser Cys Ser Asp Leu Glu Ser Met Lys Thr Ser Cys Ser His Gly Ser
            735                 740                 745

agt gat tat cac acc cgc ttt gct act gtt aac att ctt cct gag gta 2606
Ser Asp Tyr His Thr Arg Phe Ala Thr Val Asn Ile Leu Pro Glu Val
        750                 755                 760

gaa aat gac cgt ctg gaa aat tcc cca cat cag tgt agc att tct gtg 2654
Glu Asn Asp Arg Leu Glu Asn Ser Pro His Gln Cys Ser Ile Ser Val
    765                 770                 775

gtt acc caa act gct tcc tgt tca gaa gtt tca cag ttg aat cat att 2702
Val Thr Gln Thr Ala Ser Cys Ser Glu Val Ser Gln Leu Asn His Ile
780                 785                 790                 795

gct gaa gaa cat ggt aac aat gga ata aaa cct aat gtt gat tta tat 2750
Ala Glu Glu His Gly Asn Asn Gly Ile Lys Pro Asn Val Asp Leu Tyr
                800                 805                 810

ttt ggc gat gca cta aaa gaa aca aat aac aac cac tca cat cag aca 2798
Phe Gly Asp Ala Leu Lys Glu Thr Asn Asn Asn His Ser His Gln Thr
            815                 820                 825

atg gaa tta aaa gtt gca att cag act gaa att taggcccata aatgctgcag 2851
Met Glu Leu Lys Val Ala Ile Gln Thr Glu Ile
        830                 835

aataattacc actgtacaac cgtgtttgga gctggttgaa ctacatgtga ctacttaagt 2911

ttcaggttac cagcaaaagc cgggtttcat tatcataatg cagatacatt ttctgtgttc 2971

agcaaggcat tgtgtgtcat gtggatctta gttaccaaac tatgaagtga aggctttaaa 3031

agtgcattat tttaaggata ataaatttga agagcaaagc atgttttgtg tgtttgccac 3091

aaaacattgc ttgaagcaca tacttagata gaaattggtc ttaatttata taatcaatat 3151

aaaatactaa tgcaattcta cagcattcaa atgaagaaaa cttgaggctt tagggataag 3211

tggttagtga tattttattg aaaccactaa agagataagt ttaaaagaac tgcataggtt 3271

actctcagta tatgatactc tgtaacattt ctatttatat cggcataaat ttcatttttt 3331

ttcttcatat gcaatgtggt tatataaagc ttaatgcagc tcatttgcta ccatttggat 3391

acttagacac tttgagcaag attgtggcag tttttgcaca actttgaaat agaaatacct 3451

ggtactctat cttgtttatt gttgatgcca tcttagagga aaaaatgtaa aggtaagtaa 3511

ttaagcatat gacagcaaca aataagatac ataaaactac aaaataaagt cccattaggt 3571

tataagtatt acaaaaaatc cacctttctc taaggggaag tttgtacccc attgattctt 3631

ggtgcctttg ggatcgactg ggttttaatg gcctagttat ttgaggattt tgctgtgttg 3691

ttttccatgt cttctctggt caccttggat tatatataaa aatacaggaa atagataaac 3751

atgaatgtga ttaataatgc tgaaaaagta ttagcctacc aaagacacac tcaggcttta 3811

gtgaataact ttacataacc tcagttttta acacatgcat atcttctcca accatgaaat 3871

caaagcacgg tgcagaactt gtaccaagta caaaaggtcc atgtatgatt agcattattt 3931

tcttttgctt ttgtttatgg acaatgttca gctgacataa gcagaagttg gccaaaatac 3991

tgcctgtact gttaatttcc tgtataattc acttaaataa aagcaggtta acctcaatga 4051

tagcagttaa aatgttctat cttatgtatt tcttttaagt attaccatta tggtgctact 4111

gagcgttttc ttttggtaaa aagaaaaatg ccatgggctg cagtcttctt ccatcacttt 4171

tccctaccag gtccattaat atgcttataa cactagtgcc agttatttta tttgataatg 4231

cttatggtat ttgtatattt gtttgcattc caattttgtt taataatgag tgtgtaaact 4291

gcatacgtta aataaatgta aatactaatg tactgctgca aaaaaaaaaa aaaaaaaaaa 4351

aaaaaa 4357

<210> 4
<211> 1780
<212> DNA
<213> Homo sapiens

<220>
<221> CDS
<222> (676)..(1332)

<400> 4
acttccgctg ctrtttcgta gccgactgct gaaggctggt ttgcgtcgac atggcggtta 60

ccctgagtct cttgctgggc gggcgcgttt gcgccgccgt cactcgctgt gggttcgcga 120

cccggggggt ggcgggccca ggccctattg gccgggagcc ggaccccgat tccgactggg 180

agccggagaa cgggagctgc aggaggtgga gaggtaccgg cttctccccg ggccctcagc 240

ttgaagcagg gcctcgtgct ccggcgctcc aggccgcgcc cccttggcgc cgggtgtcct 300

gccgctgctt gcgcagcggc ccttgtttct tcttacccgt tgttaggggc gcacgtcagg 360

tgttcagctc ccctgggacc actggtcctt tcattagtga cgtatttcat catcagttta 420

gagagttcgg catgcttaca ggcagttatt gttctaggtg ttagctttct gggtgtacgg 480

agcagctcta agccggcaac atggcccggt tgcccttgcg atcaaagaga agagggctgg 540

gcgctccatg atttagcctg aggctcttca aacatccatt ctgcttccac gcatggcttc 600

tgccattggt tctcttcccc cagcaccctg aaacgacaga aacaagcaat ccgattccag 660

aaaattcggt ggcaa atg gag gcg cct ggt gcc ccg ccc agg acc ctg acg 711
Met Glu Ala Pro Gly Ala Pro Pro Arg Thr Leu Thr
1               5                   10

tgg gaa gcc atg gag cag ata cgg tat tta cat gag gaa ttt cca gag 759
Trp Glu Ala Met Glu Gln Ile Arg Tyr Leu His Glu Glu Phe Pro Glu
        15                  20                  25

tcc tgg tca gtt ccc agg ttg gct gaa ggc ttt gat gtc agc act gat 807
Ser Trp Ser Val Pro Arg Leu Ala Glu Gly Phe Asp Val Ser Thr Asp
    30                  35                  40

gtg atc cga aga gtt tta aaa agc aag ttt tta ccc aca ttg gag cag 855
Val Ile Arg Arg Val Leu Lys Ser Lys Phe Leu Pro Thr Leu Glu Gln
45                  50                  55                  60

aag ctg aag cag gat caa aaa gtc ctt aag aaa gct ggg ctt gcc cac 903
Lys Leu Lys Gln Asp Gln Lys Val Leu Lys Lys Ala Gly Leu Ala His
                65                  70                  75

tcg ctg cag cac ctc cgg ggc tct gga aat acc tca aag ctg ctc cct 951
Ser Leu Gln His Leu Arg Gly Ser Gly Asn Thr Ser Lys Leu Leu Pro
            80                  85                  90

gca ggc cac tct gta tca ggc tct ttg ctt atg cca ggg cat gaa gcc 999
Ala Gly His Ser Val Ser Gly Ser Leu Leu Met Pro Gly His Glu Ala
        95                  100                 105

tca tct aaa gac cca aat cac agc aca gct ttg aaa gtg ata gag tca 1047
Ser Ser Lys Asp Pro Asn His Ser Thr Ala Leu Lys Val Ile Glu Ser
    110                 115                 120

gac act cac agg aca aat aca cca agg aga agg aag gga aga aat aaa 1095
Asp Thr His Arg Thr Asn Thr Pro Arg Arg Arg Lys Gly Arg Asn Lys
125                 130                 135                 140

gaa atc cag gac ctg gag gag agc ttt gtg cct gtt gct gca ccc cta 1143
Glu Ile Gln Asp Leu Glu Glu Ser Phe Val Pro Val Ala Ala Pro Leu
                145                 150                 155

ggt cat cca aga gag ctg cag aag tac tcc agt gat tct gag agc ccc 1191
Gly His Pro Arg Glu Leu Gln Lys Tyr Ser Ser Asp Ser Glu Ser Pro
            160                 165                 170

aga gga act ggc agt ggt gcg ttg cca agt ggt cag aag ctg gag gag 1239
Arg Gly Thr Gly Ser Gly Ala Leu Pro Ser Gly Gln Lys Leu Glu Glu
        175                 180                 185

ttg aag gca gag gag cca gat aac ttc agc agc aaa gta gtg cag agg 1287
Leu Lys Ala Glu Glu Pro Asp Asn Phe Ser Ser Lys Val Val Gln Arg
    190                 195                 200

ggc cga gag ttc ttt gac agc aac ggg aac ttc ctg tac aga att 1332
Gly Arg Glu Phe Phe Asp Ser Asn Gly Asn Phe Leu Tyr Arg Ile
205                 210                 215

tgagtcgggg cttggcttat ggagatgcct cgtgaaacac agctgggcaa gtattaatgt 1392

atatggaaca gcctggattt ctgcatatgg ataagccacc ttggaatagg aagaggtgtt 1452

gagcctggac tgtgggagga aagagctgcg tggatagatt caaacttcct gtggtagtgc 1512

tcccagtctg acctctgtag accttcagta ctcactcttc ttgcttaggc tctctgtgtg 1572

ttgaaagcca tcccgtgttg catgtgttgt tacaattttc tgtgatactt gcaatttatg 1632

tttgagaaga agtgaaaagt ttgccttctg acctcatttc cttcttgatc agtgaacact 1692

aacattttgg ggacaactta gtcaattggt tttccttaca acaaaataaa gtaaaatgta 1752

gcagtcaaaa aaaaaaaaaa aaaaaaaa 1780

<210> 5
<211> 21
<212> DNA
<213> Artificial Sequence

<223> Description of Artificial Sequence:synthetic DNA

<400> 5
tcaatcgtca tcgatcagcc c 21

<210> 6
<211> 25
<212> DNA
<213> Artificial Sequence

<223> Description of Artificial Sequence:synthetic DNA

<400> 6
nnnngggctg atcgatgacg attga 25

## Description

### Technical Field

The present invention relates to novel proteins and genes thereof. More specifically, the present invention relates to proteins involved in amyotrophic lateral sclerosis, and genes encoding these proteins.

### Background Art

Amyotrophic lateral sclerosis (hereinafter referred to as "ALS") is a neuro-degenerative disease estimated to afflict about 5,000 people in Japan, in which motor neurocytes atrophy and decrease in number, and accompanying this, the muscles controlled by these nerves atrophy. ALS is a disease whose cause is unknown. However, if a factor which specifies the disease is found, it is thought that this factor per se or an inhibitor thereof can be a therapeutic agent against the disease. From this standpoint, identification of this factor is desired, but there is as yet no report that the factor has been cloned.

### Disclosure of the Invention

The present inventors have purified RNA from the spinal marrow of ALS patients and normal individuals, and have isolated mRNA which specifically increases or decreases in ALS patients by using a molecular indexing. By cloning genes from these mRNA and conducting further examination, the present invention has been completed.

Thus, the present invention provides the following:
(1) A protein having an amino acid sequence shown in SEQ ID NO:1 or SEQ ID NO:2, or an amino acid sequence having deletion, substitution or insertion of one or plural amino acids in said amino acid sequences; or a salt thereof.
(2) The protein or salt thereof according to claim 1, which is characterized in that its expression increases in an amyotrophic lateral sclerosis patient.
(3) A gene comprising the nucleotide sequence shown in SEQ ID NO: 3 or SEQ ID NO: 4, or a gene which hybridizes with said gene under stringent conditions and encodes a protein, the expression of which increases in an amyotrophic lateral sclerosis patient.
(4) A gene encoding the protein of claim 1.
(5) An expression vector comprising the gene of claim 3 or 4.
(6) A transformant transformed with the expression vector of claim 5.
(7) A partial peptide of the protein of claim 1 or 2; or a salt thereof.
(8) An antibody against any of the protein of claim 1 or 2 or the partial peptide of claim 7.
(9) A method for screening a compound for treatment of a neuro-degenerative disease, preferably treatment of amyotrophic lateral sclerosis, which is characterized in that the protein of claim 1 or 2 or the partial peptide of claim 7 is used.
(10) A kit for the screening of a compounds for treatment of a neuro-degenerative disease, preferably treatment of amyotrophic lateral sclerosis, which is characterized in that the protein of claim 1 or 2 or the partial peptide of claim 7 is used.
(11) A transgenic animal, wherein the gene of claim 3 or 4 is artificially introduced into a chromosome, or either gene is deleted from a chromosome.

The proteins and genes of the present invention shows an expression or an inhibition of expression, which is specific to ALS patient, and therefore are thought to be involved in the cause and pathology of ALS. Therefore, there is a possibility that the progress of the disease can be halted and the disease can be directed toward improvement by inhibiting or increasing the expression or function of these proteins. That is, these proteins may be a target of therapeutic agents for ALS. Further, since these proteins play ALS patient-specifically, there is a possibility that these proteins become a target protein for easy diagnosis of ALS for which there is no decisive method of diagnosis at present.

Further, the mechanism of onset of most diseases of the neuro-degenerative diseases including ALS is unknown. However, since there is a possibility that they have a common mechanism which finally lead neurocytes to death, there is a possibility that the proteins and genes of the present invention become a target of general therapeutic agents for neuro-degenerative diseases.

### Best Mode for Carrying out the Invention

The present invention will be explained in detail below.

The protein of the present invention is a protein having an amino acid sequence which is identical or substantially identical to the amino acid sequence shown by SEQ ID NO: 1 or SEQ ID NO: 2. The protein having an amino acid sequence which is substantially identical to the amino acid sequence shown by by SEQ ID NO: 1 or SEQ ID NO: 2 is one which has 70% or more, preferably 80% or more, more preferably 90% or more, or most preferably 95% or more identity to the amino acid sequence of SEQ ID NO: 1 or SEQ ID NO: 2, and either which is expressed specifically in ALS patients or the expression of which is inhibited specifically in ALS patients. The expression can be changed depending on the amino acid sequence of the protein. However, to the extent that such expression or inhibition of expression is ALS patient-specific, the amino acid sequence may be changed. Specifically, the protein of the present invention includes fragments or analogs of the proteins having an amino acid sequence which has deletion, substitution or insertion of one or plural, preferably 1 to 10, more preferably 1 to 5, or further preferably, 1, 2, 3 or 4 amino acids in the amino acid sequences of SEQ ID NO: 1 or SEQ ID NO: 2. The fragments and analogs can be obtained by introducing deletion, substitution or insertion of amino acid sequences at a site which is not important for the function of the protein of the present invention. Amino acids at a site which is important for the function of the protein cannot be deleted or substituted. Also, it is not possible to alter sites involved in conservation of the overall structure of the protein in connection with its biological activity, by introducing deletion, substitution or insertion of amino acids. Amino acids which can be substituted are ones having a similar size or polarity. Examples thereof include mutual substitution between aliphatic amino acids, Ala, Val, Leu and Ile, mutual substitution between Ser and Thr which have hydroxyl group, mutual substitution between Asp and Glu which have acidic residue, substitution between Asn and Gin which have amide residue, substitution between Lys and Arg which have basic residue, and substitution between Phe and Tyr which have aromatic residue.

A precursor of the protein of the present invention is also encompassed within the protein of the present invention, so long as it has the above described biological activity. Examples of such precursors include peptides of the present invention to which one or more amino acids are added to the N terminal side and/or C terminal side.

A physiologically acceptable salt of the protein of the present invention or a precursor thereof, is also encompassed by the present invention. Examples of such a salt include acid addition salts including salts of inorganic acids such as hydrochloric acid, phosphoric acid or sulfuric acid, and salts of organic acids such as acetic acid, formic acid, fumaric acid, maleic acid, succinic acid, citric acid, tartaric acid, malic acid, benzoic acid, or benzenesulfonic acid.

The gene of the present invention includes a gene which encodes the above-mentioned protein, and specifically includes a gene having a nucleotide sequence shown by SEQ ID NO: 3, which is a gene encoding a protein having the amino acid sequence shown by SEQ ID NO: 1, or SEQ ID NO:4, which is a gene encoding a protein having the amino acid sequence shown by SEQ ID NO: 2. Further, a gene which hybridizes under stringent conditions with a gene comprising the nucleotide sequence of SEQ ID NO: 3 or SEQ ID NO: 4, is encompassed within the present invention.

Herein, "a gene which hybridizes under stringent conditions" refers to, for example, a gene which hybridizes with a gene comprising the nucleotide sequence of SEQ ID NO: 3 or SEQ ID NO: 4 under conditions typically used for "probe hybridization." These genes are cloned by hybridization with the gene of SEQ ID NO: 3 or SEQ ID NO: 4. Specific procedures such as cDNA library preparation, hybridization, positive colony selection, determination of nucleotide sequence, are known, and can be easily performed by reference to, for example, *Molecular Cloning* 2^{nd} Edt. Cold Spring Harbor Laboratory Press (1989).

In the present invention, a partial peptide refers to a partial peptide composed of at least 6 amino acids among the amino acid sequence of the protein of the present invention. Herein, the limitation of "at least 6 amino acids" is due to the fact that 6 amino acids is the minimum size which can achieve a stable structure. A peptide having a length of 8 amino acids or more is preferred, and a peptide having a length of approximately 10 to 20 amino acids is more preferred. If the partial peptide is a short peptide of about 10 to 20 amino acids, it can be easily synthesized with a peptide synthesizer. If the partial peptide is long, it can be obtained by expressing the peptide prepared according to ordinary gene engineering techniques (for example, by treatment with restriction enzymes, etc.) in animal cells and the like. It is possible to modify a peptide prepared in this manner by ordinary techniques. These partial peptides can be used for the preparation of antibodies as described later.

The protein and gene of the present invention will be explained below.
1. Collection of Samples
   Spinal cords are collected by surgical methods from the lumbar region of a deceased ALS patient, and also a person having a normal lumbar region who died of a cause other than ALS, in as short a time as possible after death was confirmed.
2. Purification of RNA
   Purification of RNA from spinal cords can be performed, for example, by using TRIzol TM (GIBCO-BRL) according to the attached protocol.
3. Molecular Indexing
   Molecular Indexing using the obtained RNA can be performed, for example, according to the method of K. Kato (Nucleic Acids Research 23: 3685 1995).
   1) Synthesis of double stranded cDNA
      Using an oligo dT primer, cDNA is synthesized from RNA with reverse transcriptase. Further, a double stranded cDNA is synthesized using DNA polymerase I.
   2) Cleavage with Class IIS Restriction Enzymes
      Next, cDNA is cleaved respectively with three types of class IIS restriction enzyme. For example, Fok II, BsmA I and BsmF I can be used. Class IIS restriction enzymes are restriction enzymes which cleave a site detached by a certain distance from the recognition site, and produce a sticky end having random sequence at the cleaved end. Three groups are produced for each restriction enzyme.
   3) Linking of biotinated adapter
      64 types of biotinated adapter having 3'-end protruding ends corresponding to the 64 permutations of 4-base sticky ends produced in 2) above, are synthesized. Each adapter is linked to cDNA having a matching sequence using *E. coli* DNA ligase, concentrated with streptavidin coated magnetic beads, and collected. After adapter ligation, cDNA is treated with two types of class IIS restriction enzymes other than the enzymes used in the initial cleavage. Thereby, only cDNA wherein the recognition sites of the initially used class IIS restriction enzyme are most proximate to the poly A tail, is selected. In combination with 2), the cDNA are divided into 192 groups.
   4) PCR
      After cleaving with restriction enzymes, the double stranded cDNA is alkali denatured using 0.1N NaOH to form single strands. Thereafter, using a primer set of a sense primer which is complementary to the adapter common sequence, and 3 types of antisense anchor oligo dT primer to which are linked A, C or G being complementary to T, G or C as the bases proximate to the poly A tail, PCR is performed for a further division into 3 for each of the adapter-linked groups, to produce 576 types of PCR product.
   5) Polyacrylamide electrophoresis
      The 576 types of PCR product obtained in 4) above are run on a denaturing polyacrylamide gel. Fragment analysis is performed using an auto sequencer, and upon comparison of a person having a normal lumbar region and ALS patient, cDNAs are selected which show increased expression that is ALS patient specific.
4. Full-length cDNA cloning
   Using the partial cDNA selected in 3) to 5) as a probe, screening of a commercially available cDNA library is performed, and sequences of the obtained positive colonies are determined by an ordinary method. Further, using mRNA purified with an oligo dT column from RNA obtained in 1, the 5' end is elongated by 5' RACE (Rapid Amplification of cDNA Ends) to obtain the beginning of the open reading frame. The sequence of the elongated cDNA is determined by an ordinary method.

Using the thus obtained gene encoding the protein of the present invention, it is possible by gene recombinant techniques to produce the protein in a large scale and use it for medical uses and the like.

That is, a prokaryotic or eukaryotic host cell can be transformed by incorporating the gene encoding the protein of the present invention into a suitable vector. The expression vector may also contain a replication origin, a selective marker, a promoter, an RNA splice site, a polyadenylation signal and the like.

Further, by introducing a suitable promoter or a sequence involved in trait expression into these vectors, the gene can be expressed in the respective host cell. Further, by ligating a gene encoding another polypeptide to a gene of interest to enable expression as a fusion protein, it is possible to simplify purification, increase the amount of expression, and by performing suitable processing in the purification step, excise a protein of interest.

Among hosts to be used in an expression system, examples of prokaryotic organism host cells include *E. coli, Bacillus subtilis* and the like. Further, among eukaryotic organisms, eukaryotic microorganism host cells include yeast, slime fungus and the like. Insect cells such as Sf9 may be used as a host cell. Further, examples of animal cell-derived host cells include COS cells, CHO cells and the like.

The proteins which was produced by culturing the transformant transformed with the gene encoding the protein of the present invention as mentioned above, can be isolated intracellularly or extracellularly and can be purified. Ordinary methods used for isolating and purifying a protein can be used for isolation and purification of the protein. For example, various types of chromatography, ultrafiltration, salting out, dialysis and the like can be selected and combined as appropriate.

In the present invention, an antibody is an antibody against either the protein of the present invention, or its partial peptide which can constitute an epitope. The antibody can be easily produced by immunizing an animal such as rabbit by normal methods such as the method described in *Shinsaibokogakujikken Protocol* p 210 Shujunsha (1993); Shin Seikagaku Jikken Koza 1, *Tanpakushitu I*, p.389-397 (1992), and the like. Antibodies include polyclonal antibodies and monoclonal antibodies, and methods for producing them are known to those skilled in the art. For example, monoclonal antibodies can easily be produced by using the method described in Protein Experimental Methods for Molecular Biological Research Chapter 4, Yodosha (1994). Further, pseudo-humanized antibodies can be obtained according to Japanese Patent Application Laying-Open (Kokai) No. 62-296890. These antibodies can be used as diagnostic agents and experimental reagents used in various immunological assay methods including enzyme immunoassay such as ELISA, radio immunoassay and immunofluorescent methods, and also can be used in affinity chromatography, screening of cDNA library, and the like.

The protein of the present invention can be used in the screening of a medicament for treatment of a neuro-degenerative disease, preferably ALS. That is, under conditions where a candidate compound for the medicament and the protein or partial peptide of the present invention can interact with each other , and in the presence of a second component which can provide a detectable signal depending on the interaction between the candidate compound and the protein or partial peptide of the present invention, the candidate compound and the protein or partial peptide of the present invention are allowed to interact, and by detecting the presence or absence of a signal produced by the interaction, a determination can be made as to whether or not the candidate compound inhibits or activates the activity of the protein or partial peptide of the present invention.

In the present invention, a transgenic animal refers to what is known as a transgenic animal where the gene of the present invention has been artificially introduced into a chromosome of an animal other than a human, and what is known as a knock-out animal where the gene has been deleted from a chromosome thereof. These transgenic animals can be easily produced by those skilled in the art based on known methods such as one regarding a disease model mouse (e.g. Molecular Medicine - Interim Edition, Nakayama Shoten (1994)). The transgenic animal is extremely useful as a model animal for the development of therapeutic agents for a neuro-degenerative disease, preferably, a therapeutic agent for ALS, and as a animal for screening for such therapeutic agents.

### Examples

The present invention will be explained in more detail below by way of Examples. However, the present invention is not limited by the following Examples so long as the present invention does not exceed the gist. Except where otherwise specified, the procedures in the following Examples were performed according to methods I-V below.
I. Cleavage of DNA with restriction enzymes was carried out with commercially available restriction enzymes using a buffer solution of a composition as indicated on the product, by reacting 2 units of enzyme per 1µg of DNA at 37°C for 1 hour.
II. Transformation of *E. coli* was performed according to the method in *Molecular Cloning* (1982) p249, Cold Spring Harbor Labs. Purification of plasmids from *E. coli* was performed by the method described on p86 thereof.
III. Ligation of DNA by T4 DNA ligase was performed as follows. Two fragments to be ligated were dissolved in a ligation buffer [comprising 66mM Tris-HCl (pH 7.5), 6.6mM magnesium chloride, and 10mM dithiothreitol] to 1µg/10µl, and 66µM of ATP was added, and then T4 ligase was added to a concentration of 0.1 units/µg DNA. Reaction was performed at 4°C for 18 hours.
IV. For DNA purification from the DNA solution, the following procedures were performed. To a solution containing DNA such as a reaction solution, an equivalent volume of phenol/chloroform was added, and the mixture was mixed vigorously. Then, phenol/chloroform extraction was performed by a centrifugation to collect the aqueous phase containing the nucleic acid.
   To the thus obtained aqueous phase, one tenth volume of 3M sodium acetate or an equivalent volume of 4M ammonium acetate, and twice (to the volume of aqueous phase) volume of ethanol were added and mixed. After allowing the mixture to stand overnight at -20°C, or for more than 15 minutes at -80°C, the mixture was centrifuged at 15,000 revolutions, and the nucleic acid was collected as a precipitate, i.e., ethanol precipitation was performed.
V. Excision of DNA from gel was performed by adding elution solution (500mM NH₄OAc, 10mM MgCl₂, 1mM EDTA, 0.1% SDS), reacting at 37°C for 2 hours, collecting the supernatant, and thereafter performing the method of IV.

### Example 1

1 Collection of Sample
   Autopsy was performed within 2 hours after confirmation of death, and the spinal cord was frozen with liquid nitrogen immediately after collection. Only the anterior of the spinal cord was excised with a sterilized scalpel, and purification of total RNA was performed with TRIzolTM (GIBCO-BRL) according to the attached protocol.
2 Molecular Indexing
   From 6µg of the purified total RNA, cDNA was synthesized using an oligo dT primer by using RT-PCR kit ver 2.1 (TAKARA) in accordance with the attached protocol. This cDNA was purified according to IV above, and dissolved in 10µl of sterilized water. To this was added 1 unit of DNA polymerase I, and double stranded cDNA was synthesized by reacting at 37°C for 1 hour in a total 20µl system. This double stranded cDNA was purified according to IV above, and dissolved in 10µl of sterilized water. Next, cDNA was respectively cleaved with 3 types of class IIS restriction enzyme, Fok II, BsmA I, BsmF I. These were then purified according to IV above and dissolved in 10µl of sterilized water. Separately, 64 types of biotinated adapter ([Table 1] SEQ ID NO: 5, 6) were synthesized (AMERSHAM-PHARMACIA), 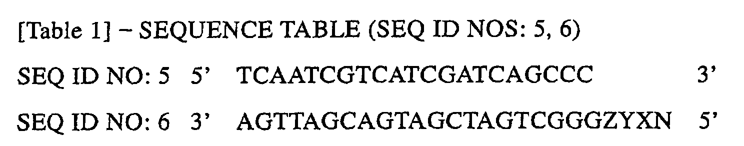 In SEQ ID NO: 6 of [Table 1], N represents any nucleotide. Z, X and Y each represent four types of nucleotide, and from the combinations which can be adopted, there are 64 types of SEQ ID NO: 6.
   Each biotinated adapter and cDNA cleaved with a restriction enzyme were ligated according to the method of III above to obtain 192 types of double stranded cDNA. These were purified according to IV above and were dissolved in 10µl of sterilized water. Next, 100µl of streptoavidin coated magnetic beads (GIBCO-BRL) was added, and allowed to react at room temperature for 1 hour. Streptoavidin coated magnetic beads were adsorbed to magnet, and were washed three times with 1ml of 100mM Tris-HCl (pH 7.5) to remove non-specifically adsorbed cDNA. Next, cDNA was treated according to I above with two class IIS restriction enzymes other than the enzymes used in the initial cleaving. These were purified according to IV above, and dissolved in 10µl of sterilized water. 10µl of 0.2N NaOH was added to the double stranded cDNA, and reacted at room temperature for 10 minutes to form single stranded cDNA. These were purified according to IV above, and dissolved in 10µl of sterilized water. Using these as a template, the following PCR was performed. Using a primer set of a sense primer (SEQ ID NO: 5) which is complementary to the adapter common sequence labeled with Texas Red, and 3 types of antisense anchor oligo dT primer to which are linked A, C or G being complementary to T, G or C as the base proximate to the poly A tail, PCRs were performed for a further 3 groups for each of the adapter-linked groups.
   PCR was performed in a 50 µl system using 1 unit of Taq polymerase (Perkins Elmer), by heating at 94°C for 5 minutes; performing 30 cycles of 30 seconds at 94°C, 30 seconds at 55°C, and 30 seconds at 72°C; and then heating at 72°C for 5 minutes. Thereby, 576 types of PCR product were obtained.
3 Polyacrylamide Electrophoresis
   The PCR products obtained in 2 above were run on a 5% denaturing polyacrylamide gel using an auto sequencer (HITACHI). Analysis was performed with SQ5500 (HITACHI), and cDNA having a greatly different expression ratio, BsmFI/39A (133bp) was selected. The selected cDNA was excised from the gel according V above, amplified again by PCR under the same condition, purified with a PCR purification kit (QIAGEN) in accordance with the attached protocol, and then dissolved in 50 µl of sterilized water. 5µl of this solution was ligated with 50ng of pT7Blue vector (Novagen) using a Ligation kit (TAKARA) in accordance with the attached protocol. Using this, transformation was performed using a commercially available competent cell, DH5 (TOYOBO) according to the method described in II. According to the method of II, a plasmid was purified from a positive colony and analyzed. Using the vector primer, the cloned gene was analyzed with an auto sequencer (HITACHI).
   As a result of examination on the internet using NCBI/BLAST as to gene information of the analyzed sequence, it was found that this sequence was identical to EST, AA21375. Further, from a search of The TIGR Human Gene Index based on this sequence, it was found that this sequence was contained in THC23095.
4 Full-length cDNA cloning
   Next, using this sequence as a probe, screening of a commercially available human fatal brain cDNA library (TAKARA) was performed according to ordinary methods. An approximately 3.8kb clone could be obtained from positive colonies. Using the vector primer, the cloned gene was analyzed with an auto sequencer (HITACHI). Further, using 5' RACE system ver 2.0 (GIBCO BRL), 5' side elongation reaction was performed in accordance with the attached protocol, thereby elongating it to 4.1kb. mRNA used at this time, was the product purified using an oligo dT column (Roche) from the initially purified total RNA, 2 µg of which was used. This gene was ligated with pT7Blue vector (Novagen) using Ligation kit (TAKARA). Using this, a commercially available competent cell, DH5 (TOYOBO), was transformed according to II. Using the vector primer, the cloned gene was analyzed with an auto sequencer (HITACHI). As a result of analysis, the gene of SEQ ID NO: 3 was obtained. Further, as a result of translating this sequence, the protein of SEQ ID NO: 1 was obtained.

### Example 2

1 to 3 From Sample Collection to Polyacrylamide Electrophoresis
   576 types of PCR product obtained by performing collection of sample and molecular indexing as in Example 1 were similarly run on 5% denaturing polyacrylamide gel, and analysis was performed. BsmFI/58G (54bp) was selected as a cDNA having a greatly different expression ratio. Steps from excision of the selected cDNA from the gel to analysis of the cloned gene, were performed in a manner similar to Example 1. As a result of examination as to gene information of the analyzed sequence, this sequence was found to be identical to EST, AA15380. Further, from a search of The TIGR Human Gene Index based on this sequence, it was found that this sequence was contained in THC213488 (1748bp).
4 Full-length cDNA cloning
   Screening of a cDNA library as performed in Example 1 was not performed, and using 5' RACE system ver2.0 (GIBCO BRL), a 5' side elongation reaction was performed according to the attached protocol, thereby extending DNA by 28 bases to make 1776bp. The mRNA used at this time was the product purified using an oligo dT column (Roche) from the initially purified total RNA, 2µg of which was used. This gene was ligated with pT7Blue vector (Novagen) using Ligation kit (TAKARA). Using this, a commercially available competent cell, DH5 (TOYOBO), was transformed according to II. Using the vector primer, the cloned gene was analyzed with an auto sequencer (HITACHI). As a result of analysis, the gene of SEQ ID NO: 4 was obtained. Further, as a result of translating this sequence, the protein of SEQ ID NO: 2 was obtained.

### Industrial Applicability

The proteins and genes of the present invention shows an expression or an inhibition of expression, which is specific to ALS patient. Therefore, there is a possibility that the progress of the disease can be halted and the disease can be directed toward improvement by inhibiting or increasing the expression or function of these proteins. That is, these proteins are thought to be useful as therapeutic agents for ALS or in screening thereof. Further, an antibody against the protein of the present invention is thought to be useful in diagnosis of ALS patients.

## Claims

1. A protein having an amino acid sequence shown in SEQ ID NO:1 or SEQ ID NO:2, or an amino acid sequence having deletion, substitution or insertion of one or plural amino acids in said amino acid sequences; or a salt thereof.

2. The protein or salt thereof according to claim 1, which is **characterized in that** its expression increases in an amyotrophic lateral sclerosis patient.

3. A gene comprising the nucleotide sequence shown in SEQ ID NO: 3 or SEQ ID NO: 4, or a gene which hybridizes with said gene under stringent conditions and encodes a protein, the expression of which increases in an amyotrophic lateral sclerosis patient.

4. A gene encoding the protein of claim 1.

5. An expression vector comprising the gene of claim 3 or 4.

6. A transformant transformed with the expression vector of claim 5.

7. A partial peptide of the protein of claim 1 or 2; or a salt thereof.

8. An antibody against any of the protein of claim 1 or 2 or the partial peptide of claim 7.

9. A method for screening a compound for treatment of a neuro-degenerative disease, which is **characterized in that** the protein of claim 1 or 2 or the partial peptide of claim 7 is used.

10. The method for screening of claim 9 wherein the neuro-degenerative disease is amyotrophic lateral sclerosis.

11. A kit for the screening of a compounds for treatment of a neuro-degenerative disease, which is **characterized in that** the protein of claim 1 or 2 or the partial peptide of claim 7 is used.

12. The kit for the screening of claim 11 wherein the neuro-degenerative disease is amyotrophic lateral sclerosis.

13. A transgenic animal, wherein the gene of claim 3 or 4 is artificially introduced into a chromosome, or either gene is deleted from a chromosome.
